# EUROPEAN PATENT APPLICATION

(11) **EP 1 285 966 A1**
(43) Date of publication of application: **26.02.2003**
(21) Application number: 01119348.9
(22) Date of filing: 10.08.2001
(51) Int. Cl.: C12N 15/82, A01H 5/00

(54) **Methods and gene constructs for gene silencing in transgenic plants**

(71) Applicant: Fraunhofer-Gesellschaft, 80636 München (DE)
(72) Inventor: Wassenegger, Michael Dr., 80799 München (DE); Vogt, Ulrike, 81247 München (DE); Pütz, Arno Dr., 85221 Dachau (DE); Fischer, Rainer Prof. Dr., 52156 Monschau (DE)
(74) Representative: VOSSIUS & PARTNER

(57) **Abstract**

Described are means and methods that generally enable the specific inactivation of genes. Transgenes comprising any gene sequence, cDNA sequence or a part of them are linked to cDNA subfragments having a size of >30 bp of a viroid, here preferably potato spindle tuber viroid (PSTVd). Insertion of such constructs into expression cassettes comprising regulatory elements allow expression of the transgenes upon introduction of the constructs into plant cells. Subsequent infection of the transformed plant or plant cells with the corresponding viroid, here preferably PSTVd, results in suppression of only the transgene expression and transcribed genes that share homology with the transgene and/or the viroid sequence. Thus, the present method enables specific gene inactivation at any desired time. Examples of reporter gene inactivation are included that demonstrate the working order of the present invention.

## Description

The present invention relates to DNA constructs comprising a DNA which can be transcribed in a plant cell to an RNA transcript, which RNA transcript includes a viroid sequence linked to a target gene sequence and to recombinant vectors comprising such DNA constructs. Furthermore, the present invention relates to host cells transformed with such DNA constructs or recombinant vectors. In one particular aspect, the present invention relates to a method of providing a plant for assessing the down-regulation of expression of a target gene in a plant including the step of introducing the above described DNA construct or recombinant vector in to plant cells and to plants obtainable by said method. Furthermore, the present invention relates to a method of target gene modulation/characterization comprising infecting a plant cell or plant comprising the above described DNA construct or recombinant vector with a plant viroid. The present invention generally enables specific gene inactivation in a plant at any desired time and provides new means for specific gene inactivation and characterization of gene functions in plants.

Several documents are cited throughout the text of this specification either by name or are referred to by numerals within parenthesis. Full bibliographic citations may be found at the end of the specification immediately preceding the claims. Each of the documents cited herein (including any manufacturer's specifications, instructions, etc.) are hereby incorporated herein by reference; however, there is no admission that any document cited is indeed prior art as to the present invention.

The gene inactivation is based on a sequence-specific RNA degradation process that is induced upon infection with a viroid. The viroid infection-induced production of RNA degrading complexes is utilised to inactivate a transgene comprising a region that shares homology with the infecting viroid genome.

### Background of the invention

Post-transcriptional gene silencing (PTGS) has been found in organisms as diverse as fungi, plants, worms, flies, fish and mice. It is proposed to be a defence mechanism that uses a RNA degradation mechanism to silence gene expression. Three terms are currently used to describe gene silencing in plants (PTGS), fungi (quelling), and vertebrates or invertebrates [RNA interference (RNAi)]. However, recent findings indicate that a common mechanism probably underlies them all. All three processes are based on interactions between homologous, complementary nucleic acids and involve steps to initiate, propagate and maintain silencing (Hammond *et al*., 2001).
In plants, PTGS can be activated by viral infection or by the introduction of transgene sequences. Experiments in nematodes have shown that injection of double stranded RNA (dsRNA) into worm cells results in efficient degradation of RNAs that are complementary to the dsRNA (Fire *et al.,* 1998). Although, RNA injection experiments have not been performed with plants, it is generally accepted that dsRNA also triggers PTGS in plants. This has been supported by the demonstration that both simultaneous expression of sense and antisense transgenes (Waterhouse *et al.,* 1998) as well as transcription of transgenes arranged as inverted repeats (Stam *et al*., 1998) (IRs) are efficient triggers of PTGS.
The fact that post-transcriptional gene silencing (PTGS) is associated with *de novo* methylation in most cases indicates that DNA methylation plays an important role in the gene silencing processes (Voinnet *et al.,* 1998; Mette *et al.,* 1999; Mette *et al.,* 2000). PTGS was further demonstrated to be associated with the occurrence of 21 to 26 nt long, double-stranded RNA molecules, recently named short interfering RNAs (siRNAs) (Elbashir *et al*., 2001) that share homology with the target RNA (Hamilton and Baulcombe, 1999).

In principle there is an enormous practical potential of gene silencing for crop improvement. It is possible to silence genes conferring unwanted traits in the plant by transformation with transgene constructs containing elements of these genes. Examples of this type of application include gene silencing of ripening specific genes in tomato to improve processing and handling characteristics of the harvested fruit; gene silencing of genes involved in pollen formation so that breeders can reproducibly generate male sterile plants for the production of F1 hybrids; gene silencing of genes involved in lignin biosynthesis to facilitate paper making from vegetative tissue of the plant; gene silencing of genes involved in flower pigment production to produce novel flower colours; gene silencing of genes involved in regulatory pathways controlling development or environmental responses to produce plants with novel growth habit or (for example) disease resistance; elimination of toxic secondary metabolites by gene silencing of genes required for toxin production. In addition, gene silencing is can be useful as a means of developing virus resistant plants when the transgene is similar to a viral genome. Biosource Technologies, Inc. (Kumagai et al. (1995) WO95/34668, Kumagai et al. (1995) Proc. Natl. Acad. Sci. USA, 92, 1679-1683) have suggested the use of genetic constructions based on RNA viruses which replicate in the cytoplasm of cells to provide inhibitory RNA, either anti-sense or co-suppressor RNA. Cells are transfected with the cytoplasmically replicating genetic constructions in which the RNA encoding region is specific for the gene of interest. Similarly WO98/3608 describes the use of DNA constructs including a plant virus sequence having the ability to replicate in the cytoplasm of the plant cell for gene silencing and transgenic plants. Recently, an analogous approach has been reported by using the cDNA of the satellite RNA (satRNA) of cereal yellow dwarf luteovirus (CYDV) (Wang et al., 2001).

However, it is not clear whether virus induced gene silencing is intrinsically more reproducible than any other type of gene silencing. In fact, there is only a minority of reports that describe virus induced gene silencing. In particular, with viral transgenes the virus induced gene silencing seems to be the exception rather than the rule. A further limitation of the use of virus induced gene silencing is caused by the limitation of the host plant for approaches, where viral replication and systemic infection by the virus is required.

Thus, the technical problem underlying the present invention is to overcome one or more of the many problems associated with specific gene silencing in plants and to provide means and methods that generally enable the specific inactivation of genes.

The solution to this technical problem is achieved by providing the embodiments characterized in the claims and described herein further below.

Accordingly, the present invention relates to a DNA construct comprising a DNA which can be transcribed in a plant cell to an RNA transcript, which RNA transcript includes a viroid sequence linked to a target gene sequence.

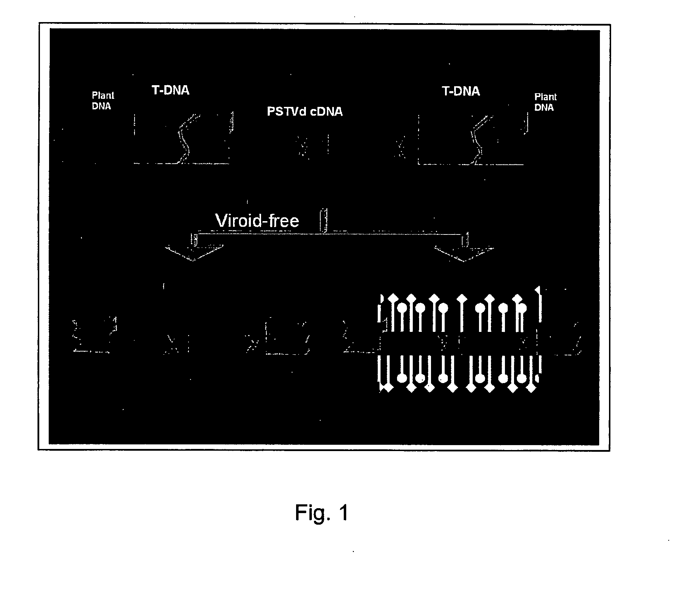

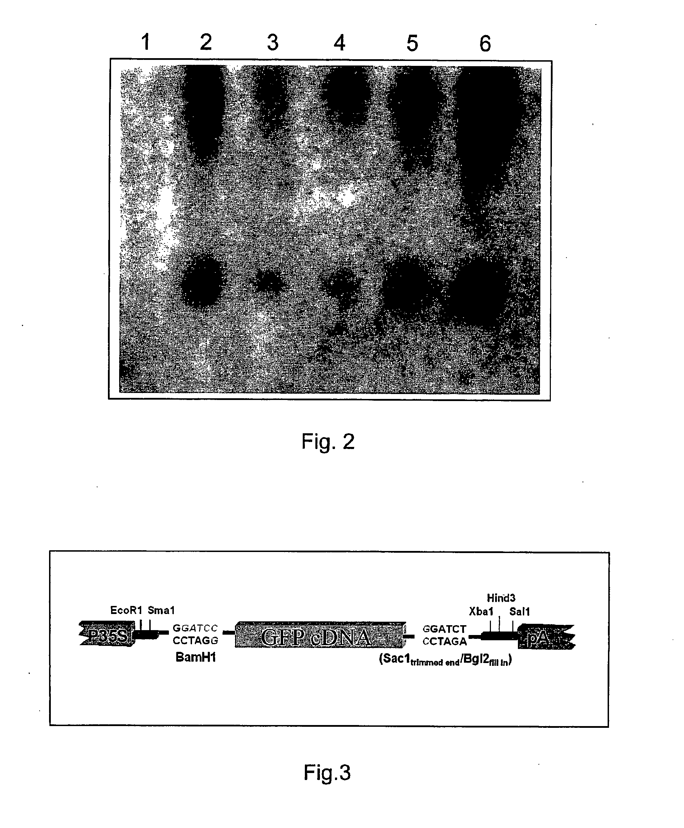

From the inventors previous work it is known that specific *de novo* methylation of plant genome-integrated potato spindle tuber viroid (PSTVd) cDNA can be triggered upon infection with PSTVd (Fig. 1) (Wassenegger *et al.,* 1994a; Pelissier *et al*., 1999; Pélissier and Wassenegger, 2000; Wassenegger, 2000). In this context it is important to note that PSTVd-specific cDNA fragments with a size of only 30 bp are targeted for methylation (Pélissier and Wassenegger, 2000). Viroids are 260-450 nt long non-encapsulated RNA pathogens that replicate via oligomeric (-)-specific RNA intermediates in the nucleus (Sänger *et al.,* 1987). Their life cycle including RNR/RNA replication, RNA processing (exception: HH viroids, see Table 1), and RNA movement is totally dependent on the host enzymes as the mature circular viroid genome does not encode a protein.
Furthermore, the inventors (see Fig. 2) and others (Papaefthimiou *et al*., 2001) demonstrated that PSTVd infection is associated with the occurrence of PSTVd-specific siRNAs. Because the presence of siRNAs is a hallmark of proceeding PTGS the inventors assumed that the plant silencing system is activated upon PSTVd infection. Based on the fact that all known viroids involve common processes to infect a plant and to propagate (see Table 1) it is to be expected that the aforementioned observations are not specific for PSTVd.

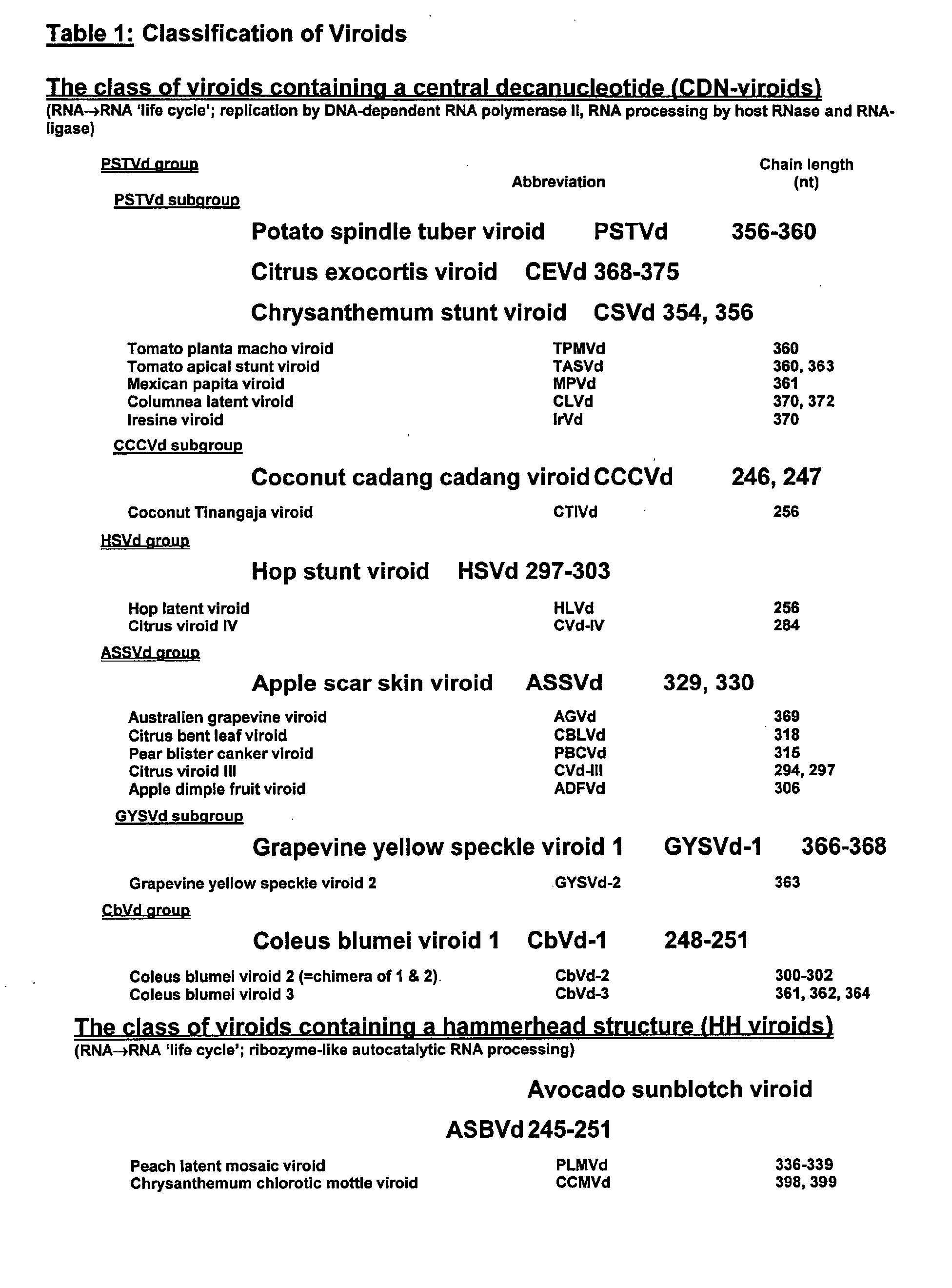

It is most likely that the RNA of all replicating viroids direct de novo methylation of complementary genomic DNA and that viroid infection generally results in induction of the plant defence mechanism.
To confirm that the occurrence of PSTVd-specific siRNAs is associated with sequence-specific RNA targeting and target RNA degradation, subgenomic cDNA fragments of the PSTVd were linked to the 3' end of a cauliflower mosaic virus 35S promoter- (P35S-) driven green fluorescent protein (GFP) gene. The resulting constructs were introduced into tobacco SR1 plants via agro-transformation. Subsequently, PSTVd infection was initiated upon mechanical inoculation of cuttings of the transgenic plants (GFP/PSTVd plants) or by genetic crosses of the GFP/PSTVd plants with plants (described in: Wassenegger et al., 1994a) carrying multiple cDNA units of the PSTVd. Imaging of the plants under UV-light revealed decreased green fluorescence in the PSTVd-infected cuttings when compared to viroid-free plants. Northern analysis demonstrated that decreased fluorescence was associated with markedly reduced levels of the GFP-specific steady state mRNA.
In order to examine whether PTGS was induced in the GFP/PSTVd plant lines, genetic crossings between GFP/PSTVd and GFP plant lines have been performed. If PTGS takes place in the GFP/PSTVd - GFP progeny, trans-inactivation of the GFP gene is expected to occur.

Thus, in accordance with the present invention it could be shown that surprisingly viroid sequences as short as 30 base pairs are sufficient to trigger gene silencing in plants when linked to a foreign target gene sequence. In a preferred embodiment of the present invention, said viroid sequence in the DNA construct has a size of at least 30 base pairs (bp).

The transcribed RNA generally includes a sequence ("targeting sequence") which is complementary to a sequence in a target gene, either in the sense or antisense orientation, or a sequence which has sufficient homology to a target sequence for down-regulation of expression of the target gene to occur. Whilst not to be bound by any theory, it is believed that sense and antisense regulation involve hybridisation between sequences which are sufficiently complementary to hybridise under conditions within a cell. The targeting sequence within the construct may be foreign to the plant viroid, i.e. of or derived from a gene or sequence which the viroid lacks.

Those skilled in the art will understand that terms such as "exogenous" or "heterologous" may equally be used in this context.

A vector which contains the construct may be used in transformation of one or more plant cells to introduce the construct stably into the genome, so that it is stably inherited from one generation to the next. This is preferably followed by regeneration of a plant from such cells to produce a transgenic plant. Thus, in further aspects, the present invention also provides the use of the construct or vector in production of a transgenic plant, methods of transformation of cells and plants, plant and microbial (particularly Agrobacterium) cells, and various plant products.

In a preferred embodiment the DNA construct comprises a promoter operably linked to said DNA. The function of the promoter if present in the DNA construct is to ensure that the DNA is transcribed into RNA containing the viroid sequences. By "promoter" is meant a sequence of nucleotides from which transcription may be initiated of DNA operably linked downstream (i.e. in the 3' direction on the sense strand of doublestranded DNA). A promoter "drives" transcription of an operably linked sequence. "Operably linked" means joined as part of the same nucleic acid molecule, suitably positioned and oriented for transcription to be initiated from the promoter. DNA operably linked to a promoter is "under transcriptional initiation regulation" of the promoter, or "in functional combination" therewith.
Preferred promoters may include the 35S promoter of cauliflower mosaic virus or the nopaline synthase promoter of Agrobacterium tumefaciens (Sanders (1987), Nucleic Acids Res., 15: 1543-1558). These promoters are expressed in many, if not all, cell types of many plants. Other constitutively expressed promoters may be used as well.

Expression comprises transcription of the heterologous DNA sequence preferably into a translatable mRNA. Regulatory elements ensuring expression in eukaryotic, i.e. plant cells are well known to those skilled in the art. In the case of eukaryotic cells they comprise optionally poly-A signals ensuring termination of transcription and stabilization of the transcript, for example, those of the 35S RNA from Cauliflower Mosaic Virus (CaMV) and the Nopaline Synthase gene from Agrobacterium tumefaciens. Additional regulatory elements may include transcriptional as well as translational enhancers. A plant translational enhancer often used is the CAMV omega sequences, the inclusion of an intron (Intron-1 from the Shrunken gene of maize, for example) has been shown to increase expression levels by up to 100-fold. (Mait, Transgenic Research 6 (1997), 143-156; Ni, Plant Journal 7 (1995), 661-676).

In accordance with the present examples, a preferred emodiment of the present invention relates to the above described DNA construct, wherein the viroid is potato spindle tuber viroid (PSTVd).

In another preferred embodiment the DNA construct of the invention comprises a target gene sequence which is a cDNA sequence or part thereof. As described herein further below, the present invention provides means and methods for specific gene inactivation. Thus, on the one hand it is possible to employ cDNA sequences encoding proteins of known function in order to simply repress of overexpress them depending on which actual strategy is intended; see also infra. On the other hand, it is also possible to employ the DNA constructs of the present invention in order to investigate cDNA sequences of unknown function or parts thereof, and to elucidate their biological activity in accordance with the further described methods of the present invention.

To obtain possible expression in all tissues of a transgenic plant, the regulatory sequences of constitutive promoters are preferably used, such as the 35 S promoter of CaMV (Odell, Nature 313 (1985), 810-812) or promoters of the polyubiquitin genes of maize (Christensen, Plant Mol. Biol. 18 (1982), 675-689). It is also immediately evident to the person skilled in the art that further regulatory elements may be added to the chimeric sequences of the invention. For example, transcriptional enhancers and/or sequences which allow for further induced expression of the promoter may be employed. Enhancer sequences functional in plants include, for example, ocs-element (Ellis, EMBO J. 6 (1987), 3203-3208); the family of ACGT-elements (hex-motif, G-box as 1-element) (Williams, Plant Cell 4 (1992), 485-496) and the cyt-1 element (Neuteboom, Plant J. 4 (1993), 525-534). In order to achieve expression in specific tissues of a transgenic plant it is possible to use tissue specific promoters (see, e.g., Stockhaus, EMBO J. 8 (1989), 2245-2251). Known are also promoters which are specifically active in tubers of potatoes or in seeds of different plants species, such as maize, Vicia, wheat, barley etc. Furthermore, the chemically inducible Tet-system may be employed (Gatz, Mol. Gen. Genet. 227 (1991); 229-237). Further suitable promoters are known to the person skilled in the art and are described, e.g., in Ward (Plant Mol. Biol. 22 (1993), 361-366). Furthermore, transcriptional terminators may be employed.

Usually, in the DNA construct of the present invention the RNA transcript lacks all or part of the viroid genome required for replication. As mentioned herein-before, and demonstrated in the examples only minor parts of viroid sequences are required in order to achieve specific gene inactivation upon virus infection. Therefore, the complete viroid genome of functional sequences required for replication are not needed in the DNA constructs of the present invention. This is also a feature which distinguishes the present invention from prior art approaches, where for example DNA constructs are used based on plant virus sequences which require the ability to replicate in the cytoplasm of the plant cell in order to achieve gene silencing. However, it is of course to be understood that the present invention also relates to DNA constructs wherein the RNA transcript comprises a functional viroid genome which is capable of replicating in the nucleus. In such an embodiment, viroid infection of the plant cell, plant tissue or plant comprising such DNA construct or corresponding recombinant vector may not be necessary in order to achieve gene inactivation because of the presence of the functional viral genome already. Nevertheless, the DNA constructs are preferred wherein the RNA transcript comprises less than the full length and functional viroid genome.

A sequence foreign to the viral nucleic acid and homologous or similar to a target gene of interest, may be included in a sense or anti-sense orientation in the DNA construct of the invention. In using anti-sense genes or partial gene sequences to down-regulate gene expression, a nucleotide sequence is placed under the control of a promoter in a "reverse orientation" such that transcription yields RNA which is complementary to normal mRNA transcribed from the "sense" strand of the target gene; see, for example, Rothstein et al, 1987; Smith et al, (1988) Nature 334, 724-726; Zhang et al, (1992) The Plant Cell 4, 1575-1588, English et al., (1996) The Plant Cell 8, 179-188. Antisense technology is also reviewed in Bourque, (1995), Plant Science 105, 125-149, and Flavell, (1994) PNAS USA 91, 3490-3496. An alternative is to use a copy of all or part of the target gene inserted in sense, that is the same, orientation as the target gene, to achieve reduction in expression of the target gene by co-suppression. See, for example, van der Krol et al., (1990) The Plant Cell 2, 291-299; Napoli et al., (1990) The Plant Cell 2, 279289; Zhang et al., (1992) The Plant Cell 4, 1575-1588, and US-A-5,231,020.

The complete sequence corresponding to the coding sequence (in reverse orientation for anti-sense) need not be used. For example fragments of sufficient length may be used. It is a routine matter for the person skilled in the art to screen fragments of various sizes and from various parts of the coding sequence to optimise the level of anti-sense inhibition. It may be advantageous to include the initiating methionine ATG codon, and perhaps one or more nucleotides upstream of the initiating codon. A further possibility is to target a conserved sequence of a gene, e.g. a sequence that is characteristic of one or more genes in one or more pathogens against which resistance is desired, such as a regulatory sequence. A foreign sequence may be 500 nucleotides or less, possibly about 400 nucleotides, about 300 nucleotides, about 200 nucleotides, or about 100 nucleotides. It may be possible to use oligonucleotides of much shorter lengths, 14-23 nucleotides, although longer fragments, and generally even longer than 500 nucleotides are preferable where possible.

It may be preferable that there is complete sequence identity in the targeting (e.g. foreign) sequence in the DNA construct and the target sequence in the plant, though total complementarity or similarity of sequence is not essential. One or more nucleotides may differ in the targeting sequence from the target gene.

Thus, a targeting sequence employed in a DNA construct in accordance with the present invention may be a wild-type sequence (e.g. gene) selected from those available, or a mutant, derivative, variant or allele, by way of insertion, addition, deletion or substitution of one or more nucleotides, of such a sequence. A foreign sequence need not include an open reading frame or specify an RNA that would be translatable. As noted, a foreign sequence may be inserted into the DNA construct in either orientation, for sense or anti-sense regulation. It may be preferred for there to be sufficient homology for the respective anti-sense and sense RNA molecules to hybridise. There may be gene silencing even where there is about 5%, 10%, 15% or 20% or more mismatch between the targeting, e.g. foreign, sequence in the DNA construct and the target gene, in the plant cell. Preferably, the homology between the target gene sequence and the sequence within the plant endogenous gene is greater than 80, 85, 90 or 95% over at least 30 bp.

The DNA construct of the present invention may comprise more than one target gene sequence. Furthermore, as mentioned above, the target gene can or can be derived from an endogenous plant gene, a transgene, or a gene from a pathogen. The term "pathogen" includes, for example, bacteria, viruses, fungi and protozoa.

In a preferred embodiment of the DNA construct of the invention said transgene encodes a (poly)peptide, cytotoxic protein, antibody, antisense RNA, sense RNA, ribozyme, transcription factor, protease, nuclease, lipase, polymerase, repressor or activator.
The antisense RNA may be a short (generally at least 10, preferably at least 14 nucleotides, and optionally up to 100 or more nucleotides) nucleotide sequence formulated to be complementary to a portion of a specific mRNA sequence and/or DNA sequence of the gene of interest. Standard methods relating to antisense technology have been described; see, e.g., Klann, Plant Physiol. 112 (1996), 1321-1330. Following transcription of the DNA sequence into antisense RNA, the antisense RNA binds to its target sequence within a cell, thereby inhibiting translation of the mRNA and down-regulating expression of the protein encoded by the mRNA. Furthermore, appropriate ribozymes can be employed (see, e.g., EP-A1 0 291 533, EP-A1 0 321 201, EP-A2 0 360 257) which specifically cleave the (pre)-mRNA of a target gene. Selection of appropriate target sites and corresponding ribozymes can be done as described for example in Steinecke, Ribozymes, Methods in Cell Biology 50, Galbraith, eds Academic Press, Inc. (1995), 449-460.
Said transcription factor can for example be a master regulatory factor that controls the expression of a cascade of genes involved in pathogen defense of the plant (Grotewold, Plant Cell 10 (1998), 721-740; Rushton and Somssich, Curr. Opin. Plant Biol. 1 (1998), 311-315). Alternatively, it can be a hybrid transcription factor containing a DNA-binding domain (e.g. of GAL4 or of the bacteriophage 434) and an activator domain (e.g. of VP16 or of any functional plant activator domain), which, when expressed in transgenic plants containing an antisense target gene, leads to specific repression (knock-out) of the desired endogenous gene function (Wilde, Plant Mol. Biol. 24 (1994), 381-388; Guyer, Genetics 149 (1998), 633-639).
Suitable lipases comprise for example phospholipases, e.g., C or A2 type phospholipases (Scherer, Plant Growth regulation 18 (1996), 125-133). Lipases are capable of releasing free fatty acids from membrane lipids, wherein these fatty acids can function as signal transducers by which general cellular defense reactions are elicited. The growing importance of free fatty acids in pathogen-defense is documented, e.g., in Scherer (1996), Roy (Plant Sci. 107 (1995), 17-25 and references cited therein) and Tavernier (Plant Sci. 104 (1995), 117-125).
Nucleases, i.e. RNases and DNases, may also be employed, of which Barnase is one candidate among others. The use of proteases in the context of this embodiment may apply to cytotoxic effects.
A signal amplification system may be constructed using polymerases. In a two-step model, an elicitor-induced polymerase, e.g., SP6-, T7- or T3-RNA polymerase, can transcribe a second recombinant gene which is controlled by a promoter to which the polymerase is highly specific. The second gene may encode for example a cytotoxic protein which is then expressed in an amplified way. A plant system based on T7-RNA polymerase was described by McBride (Proc. Natl. Acad. Sci. USA 91 (1994), 7301-7305).
Cytotoxic proteins comprise, for example, plant RIPs (ribosome inactivating proteins; (Stripe, Bio/Technology 10 (1992), 405-412), defensins (Broekaert, Plant Physiol. 108 (1995), 1353-1358), Bt toxin, α-amylase inhibitor, T4-lysozyme, avirulence gene products, or enzymes such as glucose oxidase which generate reactive oxygen species (Shah, Trends Biotechnol. 13 (1995), 362-368; Shah, Curr. Opin. Biotech. 8 (1997), 208-214; Beachy, Curr. Opin. Biotech. 8 (1997), 215-220; Cornelissen, Plant Physiol. 101 (1993), 709-712; Estruch, Nucleic Acids Res. 22 (1994), 3983-3989).

It is in principle possible to modify the coding sequence in such a way that the protein is located in any desired compartment of the plant cell. These include the nucleus, endoplasmatic reticulum, the vacuole, the mitochondria, the plastids, the apoplast, the cytoplasm etc. Methods how to carry out this modifications and signal sequences ensuring localization in a desired compartment are well known to the person skilled in the art. (Görlich, Science 271 (1996), 1513-1518; Hicks, Plant Physiol. 107 (1995), 1055-1058; Rachubinski, Cell 83 (1995), 525-528; Schatz, Science 271 (1996), 1519-1526; Schnell, Cell 83 (1995), 521-524; Verner, Science 241 (1988), 1307-1313; Vitale, BioEssays 14 (1992), 151-160).

In one preferred embodiment, the DNA construct of the invention comprises the viroid sequence link to a target gene sequence which encodes a suppressor which is capable of regulating a second transgene. This embodiment is particular suited for the construction of hypersensitive viroid inducible cell-death and high-level expression of foreign genes upon viroid infection. For example, said DNA construct with the coding sequence of the suppressor may be under the control of a constitutive promoter which ensures high-level expression of the suppressor in the plant cell. The presence of the suppressor ensures that the second transgene which is under the control of the suppressor and for example encodes a cytotoxic protein remains shut off and therefore does not harm the plant cell. Upon viroid infection the suppressor gene is inactivated so that the level of suppressor protein in the cell decreases. This in turn leads to the expression of the gene encoding the cytotoxic protein since it is no longer repressed. Thus, viroid resistance can be specifically engineered. Possible suppressor/promoter systems are known to the person skilled in the art, for example the TET-system; see supra. The mentioned system can of course also be used for the viroid inducible production of for example therapeutically important proteins or for the expression of for example antisense RNA. Several modifications of the above described strategy will be immediately evident to the person skilled in the art and are of course subject of the present invention.

In another embodiment of the DNA construct, the transgene is an endogenous plant gene and is associated with one or more of the following traits: ripening, pollen formation, lignin biosynthesis, flower pigment production, regulatory pathways controlling development, environmental responses, growth, disease resistance or toxin production.

The present invention also relates to recombinant vectors, particularly plasmids, cosmids, viruses and bacteriophages used conventionally in genetic engineering that comprise a DNA construct of the present invention. Preferably, said vector is a plant expression vector, preferably further comprising a selection marker for plants. Methods which are well known to those skilled in the art can be used to construct recombinant vectors; see, for example, the techniques described in Sambrook, Molecular Cloning A Laboratory Manual, Cold Spring Harbor Laboratory (1989) N.Y. and Ausubel, Current Protocols in Molecular Biology, Green Publishing Associates and Wiley Interscience, N.Y. (1989). Alternatively, the DNA constructs of the invention can be reconstituted into liposomes for delivery to target cells.

Advantageously, the above-described vectors of the invention comprise a selectable and/or scorable marker. Selectable marker genes useful for the selection of transformed plant cells, callus, plant tissue and plants are well known to those skilled in the art and comprise, for example, antimetabolite resistance as the basis of selection for dhfr, which confers resistance to methotrexate (Reiss, Plant Physiol. (Life Sci. Adv.) 13 (1994), 143-149); npt, which confers resistance to the aminoglycosides neomycin, kanamycin and paromycin (Herrera-Estrella, EMBO J. 2 (1983), 987-995) and hygro, which confers resistance to hygromycin (Marsh, Gene 32 (1984), 481-485). Additional selectable genes have been described, namely trpB, which allows cells to utilize indole in place of tryptophan; hisD, which allows cells to utilize histinol in place of histidine (Hartman, Proc. Natl. Acad. Sci. USA 85 (1988), 8047); mannose-6-phosphate isomerase which allows cells to utilize mannose (WO 94/20627) and ODC (ornithine decarboxylase) which confers resistance to the ornithine decarboxylase inhibitor, 2-(difluoromethyl)-DL-ornithine, DFMO (McConlogue, 1987, In: Current Communications in Molecular Biology, Cold Spring Harbor Laboratory ed.) or deaminase from Aspergillus terreus which confers resistance to Blasticidin S (Tamura, Biosci. Biotechnol. Biochem. 59 (1995), 2336-2338).
Useful scorable marker are also known to those skilled in the art and are commercially available. Advantageously, said marker is a gene encoding luciferase (Giacomin, PI. Sci. 116 (1996), 59-72; Scikantha, J. Bact. 178 (1996), 121), green fluorescent protein (Gerdes, FEBS Lett. 389 (1996), 44-47) or β-glucuronidase (Jefferson, EMBO J. 6 (1987), 3901-3907). This embodiment is particularly useful for simple and rapid screening of cells, tissues and plants containing a vector of the invention.
Preferably the recombinant vector is an Agrobacterium binary vector.

The present invention furthermore relates to host cells comprising a DNA construct or recombinant vector according to the invention.
The DNA construct or recombinant vector according to the invention which is present in the host cell may either be integrated into the genome of the host cell or it may be maintained in some form extrachromosomally. In this respect, it is also to be understood that the DNA construct or recombinant vector of the invention can be used to restore or create a mutant gene via homologous recombination (Paszkowski (ed.), Homologous Recombination and Gene Silencing in Plants. Kluwer Academic Publishers (1994)). The host cell can be any prokaryotic or eukaryotic cell, such as bacterial, insect, fungal, plant or animal cells. Preferred cells are microbial and plant cells.

In a preferred embodiment the host cell is a plant cell having incorporated into its genome the DNA construct or recombinant vector of the invention.

In a further embodiment, the present invention relates to a method of providing a plant for assessing the down-regulating expression of a target gene in a plant including the step of introducing a DNA construct or recombinant vector of the present invention into a plant cell preferably such that the construct is stably incorporated into the genome of the cell.

Methods for the introduction of foreign genes into plants are also well known in the art. These include, for example, the transformation of plant cells or tissues with T-DNA using Agrobacterium tumefaciens or Agrobacterium rhizogenes, the fusion of protoplasts, direct gene transfer (see, e.g., EP-A 164 575), injection, electroporation, vacuum infiltration, biolistic methods like particle bombardment, pollen-mediated transformation, plant RNA virus-mediated transformation, liposome-mediated transformation, transformation using wounded or enzyme-degraded immature embryos, or wounded or enzyme-degraded embryogenic callus and other methods known in the art. The vectors used in the method of the invention may contain further functional elements, for example "left border"- and "right border"-sequences of the T-DNA of Agrobacterium which allow stable integration into the plant genome. Furthermore, methods and vectors are known to the person skilled in the art which permit the generation of marker free transgenic plants, i.e. the selectable or scorable marker gene is lost at a certain stage of plant development or plant breeding. This can be achieved by, for example cotransformation (Lyznik, Plant Mol. Biol. 13 (1989), 151-161; Peng, Plant Mol. Biol. 27 (1995), 91-104) and/or by using systems which utilize enzymes capable of promoting homologous recombination in plants (see, e.g., WO97/08331; Bayley, Plant Mol. Biol. 18 (1992), 353-361); Lloyd, Mol. Gen. Genet. 242 (1994), 653-657; Maeser, Mol. Gen. Genet. 230 (1991), 170-176; Onouchi, Nucl. Acids Res. 19 (1991), 6373-6378). Methods for the preparation of appropriate vectors are described by, e.g., Sambrook (Molecular Cloning; A Laboratory Manual, 2nd Edition (1989), Cold Spring Harbor Laboratory Press, Cold Spring Harbor, NY). Suitable strains of Agrobacterium tumefaciens and vectors as well as transformation of Agrobacteria and appropriate growth and selection media are well known to those skilled in the art and are described in the prior art (GV3101 (pMK90RK), Koncz, Mol. Gen. Genet. 204 (1986), 383-396; C58C1 (pGV 3850kan), Deblaere, Nucl. Acid Res. 13 (1985), 4777; Bevan, Nucleic. Acid Res. 12(1984), 8711; Koncz, Proc. Natl. Acad. Sci. USA 86 (1989), 8467-8471; Koncz, Plant Mol. Biol. 20 (1992), 963-976; Koncz, Specialized vectors for gene tagging and expression studies. In: Plant Molecular Biology Manual Vol 2, Gelvin and Schilperoort (Eds.), Dordrecht, The Netherlands: Kluwer Academic Publ. (1994), 1-22; EP-A-120 516; Hoekema: The Binary Plant Vector System, Offsetdrukkerij Kanters B.V., Alblasserdam (1985), Chapter V, Fraley, Crit. Rev. Plant. Sci., 4, 1-46; An, EMBO J. 4 (1985), 277-287). Although the use of Agrobacterium tumefaciens is preferred in the method of the invention, other Agrobacterium strains, such as Agrobacterium rhizogenes, may be used, for example if a phenotype conferred by said strain is desired.
Methods for the transformation using biolistic methods are well known to the person skilled in the art; see, e.g., Wan, Plant Physiol. 104 (1994), 37-48; Vasil, Bio/Technology 11 (1993), 1553-1558 and Christou (1996) Trends in Plant Science 1, 423-431. Microinjection can be performed as described in Potrykus and Spangenberg (eds.), Gene Transfer To Plants. Springer Verlag, Berlin, NY (1995). The transformation of most dicotyledonous plants is possible with the methods described above. But also for the transformation of monocotyledonous plants several successful transformation techniques have been developed. These include the transformation using biolistic methods as, e.g., described above as well as protoplast transformation, electroporation of partially permeabilized cells, introduction of DNA using glass fibers, etc.

The resulting transformed plant cell can then be used to regenerate a transformed plant in a manner known by a skilled person.

Alternatively, a plant cell can be used and modified such that said plant cell expresses an endogenous gene under the control of a viroid sequence. The introduction of a viroid sequence which does not naturally control the expression of a given gene or genomic sequences using, e.g., gene targeting vectors can be done according to standard methods, see supra and, e.g., Hayashi, Science 258 (1992), 1350-1353; Fritze and Walden, Gene activation by T-DNA tagging. In Methods in Molecular biology 44 (Gartland, K.M.A. and Davey, M.R., eds). Totowa: Human Press (1995), 281-294) or transposon tagging (Chandlee, Physiologia Plantarum 78 (1990), 105-115).

In general, the plants which can be modified according to the invention can be derived from any desired plant species. They can be monocotyledonous plants or dicotyledonous plants, preferably they belong to plant species of interest in agriculture, wood culture or horticulture interest, such as crop plants (e.g. maize, rice, barley, wheat, rye, oats etc.), potatoes, oil producing plants (e.g. oilseed rape, sunflower, pea nut, soy bean, etc.), cotton, sugar beet, sugar cane, leguminous plants (e.g. beans, peas etc.), wood producing plants, preferably trees, etc.

Thus, the present invention relates also to transgenic plant cells comprising, preferably stably integrated into the genome, a DNA construct or a recombinant vector according to the invention or obtainable by the above-described method. Furthermore, the present invention also relates to transgenic plants and plant tissue comprising the above-described transgenic plant cells or obtainable by the above-described method. In a preferred embodiment of the invention, the transgenic plant is a natural host of the plant viroid from which the viroid sequence of the DNA construct is derived.

Preferably, the transgenic plant of the present invention is a cereal, a pulse, maize, wheat, potato, tapioca, rice, sorghum, millet, cassava, barley, pea, oil seed rape, sugar beet, maize, sunflower, soybean, sorghum, lettuce, endive, cabbage, onion, broccoli, cauliflower, carnation, geranium, canola, tobacco, cotton, cucurbits, carrot, strawberry, tomato, mango, peach, apple, pear, banana, melon, pepper, chrysanthemum, poplar, eucalyptus or pine.
In addition, the present invention relates to a plant which is a clone of the plant of the present invention and to a plant which is a descendant of such a plant or of any one of the above described transgenic plants of the invention.

In yet another aspect the invention also relates to harvestable parts and to propagation material of the transgenic plants according to the invention which contain transgenic plant cells described above. Harvestable parts can be in principle any useful part of a plant, for example, leaves, stems, fruit, seeds, roots etc. Propagation material includes, for example, seeds, fruits, cuttings, seedlings, tubers, rootstocks etc.

Thus, the present invention provides any clone of a transgenic plant of the present invention, seed, selfed or hybrid progeny and descendants, and any part of any of these, such as cuttings, seed. The invention provides any plant propagule, that is any part which may be used in reproduction or propagation, sexual or asexual, including cuttings, seed and so on. Also encompassed by the invention is a plant which is a sexually or asexually propagated off-spring, clone or descendant of such a plant, or any part or propagule of said plant, offspring, clone or descendant. Plant extracts and derivatives are also provided.

A further aspect of the present invention provides a method of reducing or suppressing or lowering the level of expression of a gene of interest (or "target gene") in a plant cell, the method including causing or allowing transcription from a construct as disclosed. The DNA construct may include the target gene sequence or a fragment thereof in a sense or anti-sense orientation, or a sequence with sufficient homology to the target gene sequence or a fragment thereof for the level of expression of the target gene to be reduced on production of the RNA.

With respect to the level of expression of a gene of interest in a cell, the method will generally result in a decrease in the level of expression as compared with the level in the absence of the intervention, i.e. in comparison with equivalent wild-type cells, e.g. of plants of the same species. (Cells which are wild-type in respect of the level of expression of the gene of interest may of course not be wild-type in every respect.) Where the target is a gene of a pathogen such as a virus, down-regulation of expression may provide an increase in resistance to the pathogen, particularly where the gene is required for, or is at least involved in, replication and/or infectivity.

In accordance with the above, the present invention further relates to a method of target gene modulation/characterization comprising infecting a plant cell or a transgenic plant of the present invention with a plant viroid, and optionally comparing the phenotype of said plant to that of a corresponding wild type plant. In terms of characterisation of gene functions, it is intended to link viroid-specific sequences to cDNA fragments of genes with unknown functions. Cloning of these cDNA/viroid constructs downstream of a strong plant promoter and introduction of the constructs into plants will guarantee production of cDNA/viroid-specific RNA in planta. Upon infection with the viroid suppression of the transgene and co-suppression of the homologous endogenous gene is expected. Whenever an essential gene is inactivated production of a phenotype in transformed cells will occur giving rise to the possible function of the silenced gene. Infection of the plant can be performed as known to the person skilled in the art and as described ,e.g. in the examples.
As already described above, the method of the present invention can also be used to modulate expression of transgenes, either by a gene inactivation if the transgene comprises the viroid sequence or by activation of the transgene expression if the viroid sequence in the DNA construct is linked to a master control gene which controls the expression of an endogenous gene or transgene.

In a preferred embodiment of the method of the invention said viroid is the same or similar to the viroid from which the plant viroid sequence of the DNA construct is derived. The present invention may particularly be applied in plants such as natural hosts of a plant viroid, including any mentioned herein, though it is an advantage of embodiments of the present invention that viroids may be used for gene silencing in plants which are not their natural hosts, as has been demonstrated experimentally by the inventors.

In case the above described method of the present invention is used for the characterization of DNAs of unknown function, the method preferably further comprises identifying and/or isolating the target gene of parts thereof.

For example, a random cDNA library or randomly synthesized DNA can be cloned into the DNA constructs or recombinant vectors of the present invention, and be introduced into plant cell. Transgenic plant cells or plant tissue or transgenic plants derived there from can then be infected with the corresponding viroid and looked for changes in phenotypic characteristics compared to the corresponding wild typ plant cell, plant tissue or plant.

Identification and characterization of the target gene can be done for example by sequencing of the cDNA or fragments thereof included in the DNA construct or recombinant vector of the present invention and comparing the nucleotide sequence or more preferably the encoded amino acid sequence with corresponding sequences in an appropriate database. By way of homology, it may then be possible to establish a particular function or biological activity of the identified DNA. Likewise, isolation of the target gene of part thereof can be easily performed by the person skilled in the art, for example by recloning of the recombinant vector used for the transformation of the plant. In such cases, the recombinant vector preferably comprises a bacterial selectable marker gene in order to select for bacterial transformants. In particular, genomic DNA of the transgenic plant can be isolated, digested a with restriction enzyme, religated and transformed into E. coli. The transformed E. coli strains are then plated on a selective media and transformants which harbor DNA including the original recombinant vector or part thereof can be identified. All these methods are well known to the person skilled in the art and can be easily applied to the methods of the present invention.

In view of the above, the present invention also relates to a target gene of part thereof identified and/or isolated according to any one of the above described methods of the present invention. Preferably, the DNA sequence of the target gene so identified and/or isolated was hitherto not known.

In addition, the present invention relates to a kit comprising the DNA construct or the recombinant vector of the invention. The kit of the invention may contain further ingredients such as selection markers and components for selective media suitable for the generation of transgenic plant cells, plant tissue or plants. Furthermore, the kit may include buffers and substrates for reporter genes that may be present in the DNA construct or recombinant vector of the invention.

The present invention also relates to the use of a viroid sequence as defined herein before for gene silencing or indirect gene activation in plants. "Gene silencing" is a term generally used to refer to suppression of expression of a gene. The degree of reduction may be so as to totally abolish production of the encoded gene product, but more usually the abolition of expression is partial, with some degree of expression remaining. The term should not therefore be taken to require complete "silencing" of expression. It is used herein where convenient because those skilled in the art well understand this.

As described herein before, the present invention provides means and methods for the specific gene inactivation in transgenic plants. Accodingly, the present invention generally relates to the use of a DNA construct or recombinant vector as described herein-before in the production of a transgenic plant.

These and other embodiments are disclosed and encompassed by the description and examples of the present invention. Further literature concerning any one of the methods, uses and compounds to be employed in accordance with the present invention may be retrieved from public libraries, using for example electronic devices. For example the public database "Medline" may be utilized which is available on the Internet, for example under http://www.ncbi.nlm.nih.gov/PubMed/medline.html. Further databases and addresses, such as http://www.ncbi.nlm.nih.gov/, http://www.infobiogen.fr/, http://www.fmi.ch/biology/research_tools.html, http://www.tigr.org/, are known to the person skilled in the art and can also be obtained using, e.g., http://www.lycos.com. An overview of patent information in biotechnology and a survey of relevant sources of patent information useful for retrospective searching and for current awareness is given in Berks, TIBTECH 12 (1994), 352-364.

The present invention is further described by reference to the following non-limiting figures and examples.

### The Figures show:

**Figure 1:** Schematic presentation of RNA-directed DNA methylation.
   Non-infectious PSTVd-specific cDNAs (26 bp deletion indicated by an "X" within the PSTVd cDNA) were introduced into the tobacco genome. Upon mechanical inoculation of leaves with total RNA isolated from a PSTVd-infected N. tabacum plant, PSTVd infection was initiated in several cuttings of the primary transformant. Comparison of the viroid-free and the PSTVd-infected cuttings revealed that heavy de novo methylation (white squares and circles) of the PSTVd-specific cDNA was only detectable in the PSTVd-infected cuttings (Pélissier et al., 1999).
**Figure 2:** Northern analysis to detect PSTVd-specific siRNAs.
   Total RNA from a PSTVd-free (lane1) and five PSTVd-infected tobacco SR1 plants (lane 2-6) was isolated and separated by PAGE according to Hamilton and Baulcombe (1999). The gel was blotted onto a nylon membrane and hybridized against a random primed ³²P-labled PSTVd-specific cDNA as probe. The lower hybridizing fragments represent the 21-26 nt long siRNAs.
**Figure 3:** Schematic presentation of the construction of the pPCV702SM GFP vector.
   The GFP cDNA was cut by Sac1 at the 3' end and the ends were trimmed to obtain a blunt end (Base pairs originating from the GFP cDNA are presented in Italic and grey). After end trimming the GFP cDNA was further cut by BamH1 at the 5' end. The resulting fragment (∼820 bp) was introduced into the BamH1 and the Bgl2fill in site of the binary plant transformation vector pPCV702SM. Parenthesis indicate that the restriction sites have been destroyed.

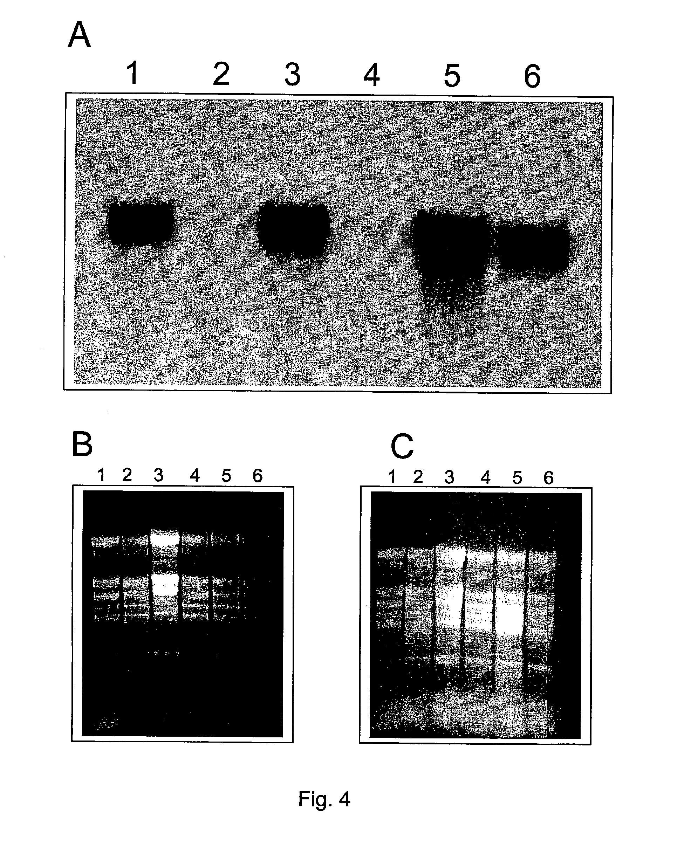

**Figure 4:** Northern analysis of PSTVd-free and PSTVd-infected SR1GFP100/1, SR1GFP100/2, and SR1GFP plant lines.
   Total RNA from the PSTVd-free (lane 1, 3, and 5) and the PSTVd-infected (lane 2, 4, and 6) SR1GFP100/1 (lane 1 and 2), SR1GFP100/2 (lane 3 and 4), and SR1GFP plant lines (lane 5 and 6) was isolated and characterized by Northern analysis as described by Sambrook et al., (1989). The gel was blotted onto a nylon membrane and hybridized against a random primed ³²P-labled GFP-specific cDNA as probe. The hybridizing fragments represent GFP-specific mRNA.
   Photograph of the ethidiumbromide-stained agarose gel to document the amount of RNA that was run on the gel.
   Photograph of the nylon membrane after blotting to document that the RNA transfer was complete.

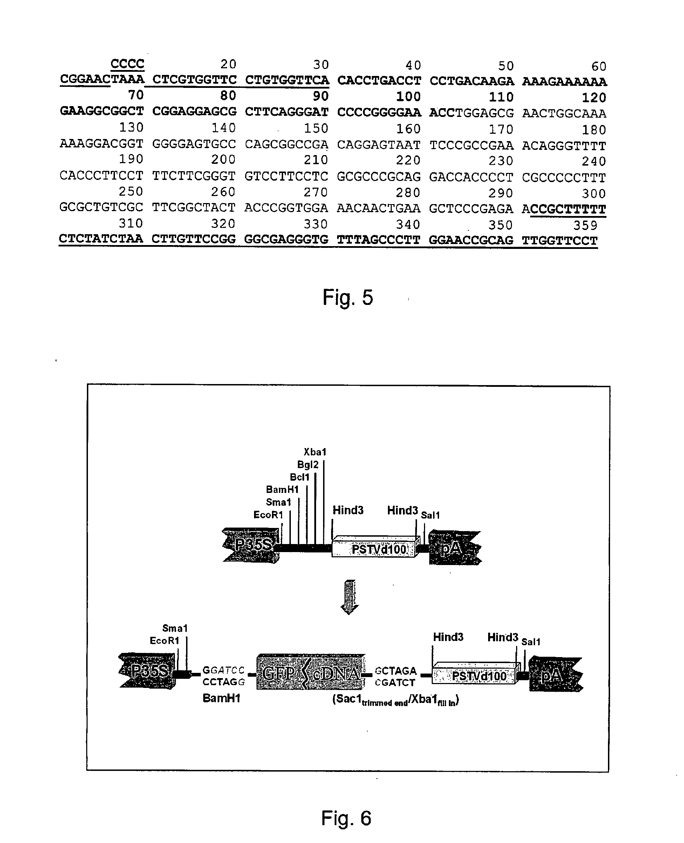

**Figure 5:** PSTVd KF440-2 cDNA sequence (Accession no.: X58388; EMBL).
   The 359 bp long cDNA sequence of the PSTVd KF440-2 type-strain is presented (SEQ ID NO: 1). The bold printed underlined sequence represents the PSTVd100 region (SEQ ID NO: 2). The bold printed sequence represents the PSTVd160 sequence (SEQ ID NO: 3). The four cytosine residues above the sequence indicate the nucleotide substitutions of the PSTVd100 sequence.
**Figure 6:** Schematic presentation of the construction of the pPCV702SM GFP100 vector.
   The PSTVd100 cDNA Hind3 fragment was cloned into the unique Hind3 site of the pPCV702SM vector in sense orientation. The recombinant plasmid was cut by Xba1 and sticky ends were filled in to produce blunt ends. The plasmid was then cut with BamH1. The GFP BamH1/Sac1trimmed end cDNA fragment was subsequently inserted into the BamH1/Xba1fill in site of the vector resulting in the pPCV702SM GFP100 plasmid.
   Base pairs originating from the GFP cDNA are presented in Italic and grey. The line within the GFP cDNA box indicates that only a part of the full length cDNA sequence is shown and parenthesis indicate that the restriction sites have been destroyed.

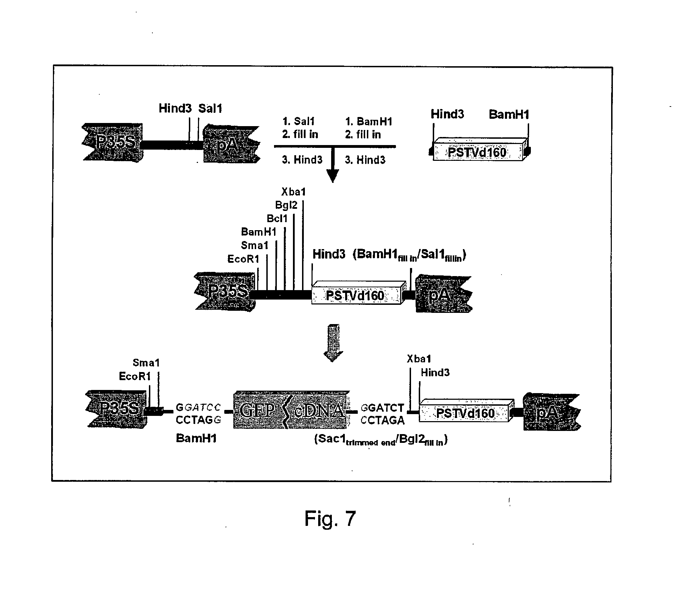

**Figure 7:** Schematic presentation of the construction of the pPCV702SM GFP160 vector.
   The PSTVd160 PCR product was cut by Bgl2 and BamH1 and introduced into the unique BamH1 site of pTPCR in sense orientation (upon cloning the Bgl2 and BamH1 site at the 5' junction was destroyed). The resulting pTPCR PSTVd160 plasmid was cut with BamH1 and sticky ends were filled in to produce blunt ends. The pPVC702SM vector was cut with Sal1 and sticky ends were filled in to produce blunt ends. Subsequently, the pPVC702SM vector was restricted with Hind3 and the PSTVd160 fragment was introduced as a Hind3/BamH1fill in fragment to give the pPCV702SM PSTVd160 plasmid. The recombinant plasmid was cut with Bgl2 and sticky ends were filled in to produce blunt ends. The plasmid was then cut with BamH1. The GFP BamH1/Sac1trimmed end cDNA fragment was subsequently inserted into the BamH1/Bgl2fill in site of the vector resulting in the pPCV702SM GFP160 plasmid.
   Base pairs originating from the GFP cDNA are presented in Italic and grey. The line within the GFP cDNA box indicates that only a part of the full length cDNA sequence is shown and parenthesis indicate that the restriction sites have been destroyed.

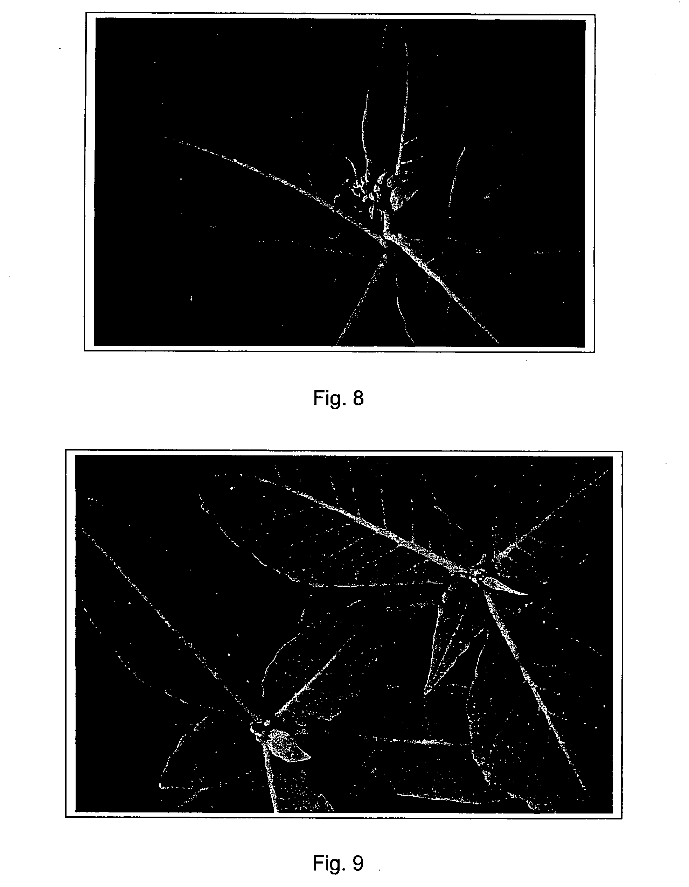

**Figure 8:** Photograph of one of the SR1GFP-4(-) progeny plants taken under UV-light.
   The plant was photographed with a NIKON COOLPIX 990 digital camera using a light-filter (Heliopan, Yellow 8, ES 62). Camera settings were: Artificial light, Matrix, Flashlight (off), ASA 400, Fine, Aperture = 2, Exposure time = 2,9", Manual focus. The picture was taken under UV-light using a handheld UV lamp (Black-Ray Long Wave UV lamp, Model B 100 AP) with a distance to the object of about 40 to 70 cm.
**Figure 9:** Photograph of a PSTVd-infected SR1GFP100-4(-) progeny plant (left) and the PSTVd-free SR1GFP100 parental plant line (right) taken under UV-light. Photographs were taken as described for figure 8.
**Figure 10:** Southern analysis of SR1GFP100, SR1GFP100-4(-), SR1GFP160, and SR1GFP160-4(-) plants.
   A: Physical map of the GFP100 (upper) and the GFP160 (lower) transgene constructs.
      All Dra1, the relevant Hpa2 sites and the resulting fragments are presented. The Hpa2 sites within the PSTVd-specific sequences are representative to demonstrate PSTVd RNA-directed de novo DNA methylation. The arrow-headed line presents the GFP cDNA that was used as probe for hybridization of the Southern blot. The size of hybridizing fragments for non-methylated DNA is 860 bp for both transgene constructs. By contrast, the fragment sizes of methylated DNA are 1000 bp for the GFP100 and 1060 bp for the GFP160 transgene construct.
      n. r. = not relevant
   B: Southern analysis.
      Southern analysis was carried out as described by Wassenegger et al. (1994a). 15 µg of Dra1/Hpa2 digested genomic DNA was separated on a 1% agarose gel, blotted (capillary) onto a (+)-charged nylon membrane, and hybridized against a random primed ³²p-labled GFP-specific cDNA as probe.
      Lane 1: genomic DNA isolated from a PSTVd-free SR1GFP100 plant line; lane 2: genomic DNA isolated from a PSTVd-infected SR1GFP100-4(-) plant line; lane 3: genomic DNA isolated from a PSTVd-free SR1GFP160 plant line; lane 4: genomic DNA isolated from a PSTVd-infected SR1GFP160 plant line; lane 5 and 6: genomic DNA isolated from two transgenic SR1 plant lines carrying unrelated pPCV702SM constructs.
      The hybridizing fragments that are indicated by arrows have sizes of about 860 bp (1), 1000 bp (2), and 1060 bp (3). Additional hybridization signals that are purely visible in lane 1 to 4 are also found in lane 5 and 6 demonstrating that either an endogenous sequence of SR1 or a sequence of the pPCV702SM vector can cross-hybridise to a low extend with the GFP probe.

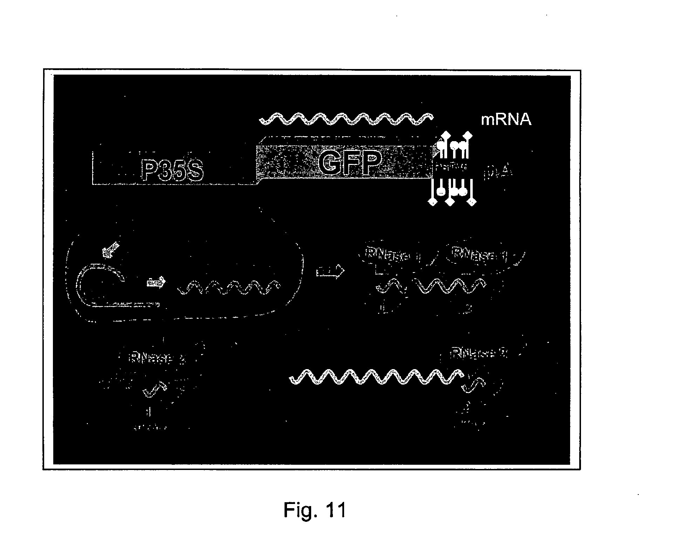

**Figure 11:** Model of methylation-independent PTGS-like processes that lead to specific degradation of the GFP100 mRNA in a PSTVd-infected SR1GFP100 plant. In the presented model a methylated GFP100 transgene (methylation is indicated by white squares and circles) is transcribed into normal, full length mRNA (GFP-specific sequences in yellow, PSTVd-specific sequences in red). The light blue ellipsoid represents the nucleus where autonomous replication of mature PSTVd (upper rod-like, circular structure) takes place. Replication proceeds according to a "rolling circle"-like mechanism resulting in double-stranded PSTVd sequence-specific RNA intermediates (plus-specific RNA in red, (-)-specific RNA in turquoise-blue). Some of the intermediates probably escape processing to finally give mature PSTVd and are instead processed to siRNAs according to Tuschl (2001). The resulting siRNAs that are complementary to the PSTVd-specific sequence of the GFP100 mRNA (siRNA 2) are capable to target the GFP100 mRNA for degradation.

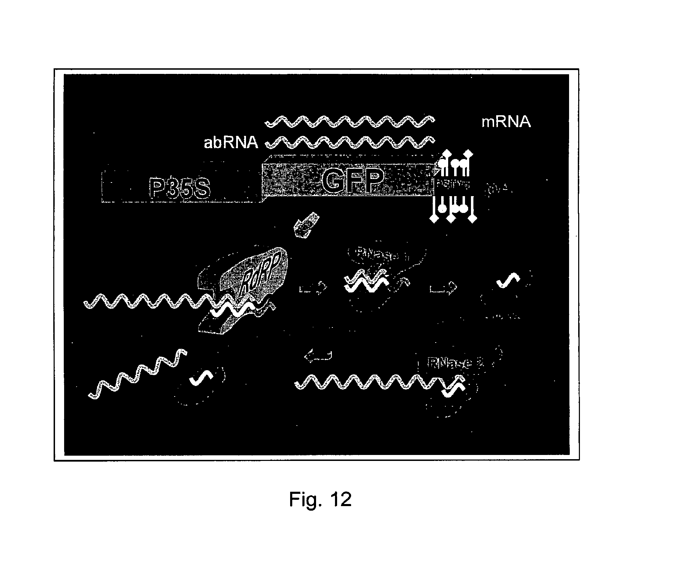

**Figure 12:** Model of methylation-dependent PTGS-like processes that lead to specific degradation of the GFP100 mRNA in a PSTVd-infected SR1GFP100 plant. In the presented model a methylated GFP100 transgene (methylation is indicated by white squares and circles) is transcribed into normal, full length mRNA and because of methylation-mediated premature termination of transcription (Voinnet et al., 1998) also into aberrant RNA (abRNA) (GFP-specific sequences in yellow, PSTVd-specific sequences in red). The abRNA is transcribed by a cellular RNA-directed RNA polymerase (RdRP) resulting in GFP sequence- and PSTVd sequence-specific dsRNA (GFP sense RNA in yellow, GFP antisense RNA in white, PSTVd plus-specific RNA in red, and PSTVd (-)-specific RNA in turquoise-blue). The dsRNA is processed to siRNAs as described in figure 11. The resulting siRNAs are complementary to GFP- and PSTVd-specific sequence and are capable to target the GFP100 mRNA for degradation.

### EXAMPLES:

Unless stated otherwise in the examples, all recombinant DNA techniques are performed according to protocols as described in Sambrook (1989), Molecular Cloning : A Laboratory Manual. Cold Spring Harbor Laboratory Press, NY or in Volumes 1 and 2 of Ausubel (1994), Current Protocols in Molecular Biology, Current Protocols. Standard materials and methods for plant molecular work are described in Plant Molecular Biology Labfase (1993) by R.D.D. Croy, jointly published by BIOS Scientific Publications Ltd. (UK) and Blackwell Scientific Publications (UK).

### Example 1: Construction of the pPCV702SM GFP vector

In order to produce a GFP-expressing control plant containing a viroid cDNA-free construct, the GFP cDNA was operatively linked to the regulatory elements of the plant transformation vector pPCV702SM (Wassenegger et al., 1994b). As shown in figure 3, a BamH1/Sac1trimmed end cDNA fragment (∼820 bp) of the GFP was inserted into the BamH1/Bgl2filled in sites of the vector. The recombinant vector (pPCV702SM GFP) was introduced into N. tabacum SR1 plants according to the leaf-disk-transformation procedure (Horsch et al., 1985). Independent transformants were regenerated, the completeness of non-rearranged T-DNA integration was shown by Southern analysis, and GFP expression was documented by taking photographs under UV-light and by Northern analysis (Fig. 4, lane 5).
Transgenic plant lines carrying a single copy of the GFP transgene constructs (SR1 GFP) were self-fertilised and progeny of this genetic crosses were screened for homozygous lines (GFP+/+) by Southern analysis. The homozygous state of the screened plant lines was confirmed by back-crosses to SR1 wild-type plants.

### Example 2: Construction of pPCV702SM GFP100 and pPCV702SM GFP160 vectors

Two GFP/PSTVd vectors were constructed by cloning into the pPCV702SM a PSTVd cDNA fragment with a size of 100 bp (PSTVd100) and a PSTVd cDNA fragment with a size of 160 bp (PSTVd160), respectively. Into these pPCV702SM PSTVd100 and pPCV702SM PSTVd160 plasmids the GFP BamH1/Sac1trimmed end cDNA fragment was inserted to give the recombinant plant transformation vectors pPCV702SM GFP100 and pPCV702SM GFP160.
The PSTVd100 cDNA fragment was amplified by PCR as described in Wassenegger (2001) from a plasmid containing the cDNA of a PSTVd mutant using the Bgl67-H forward and the PV30-H reverse primer. The PCR product was cloned into pTPCR (Wassenegger et al., 1994b). From this plasmid the PSTVd100-specific fragment was released by Hind3 and inserted into the unique Hind3 site of pPCV702SM. The PSTVd mutant carried for nucleotide substitutions (T7→C, A8→C, A9→C, and A10→C; see Fig. 5) to render possible the differentiation of PSTVd100 from the original sequence of the PSTVd KF440-2 isolate (Fig. 5). For construction of the final pPCV702SM GFP100 vector, the GFP BamH1/Sac1trimmed end cDNA fragment was ligated into the BamH1/Xba1fill in sites of pPCV702SM PSTVd100 (Fig. 6).
The PSTVd160 cDNA fragment was amplified by PCR as described in Wassenegger (2001) from a plasmid containing the cDNA of the PSTVd KF440-2 type-strain using the Bgl67-H forward and the PV175-H reverse primer. The PCR product was cut with Bgl2 and BamH1 prior to cloning into the BamH1 site of pTPCR. From this pTPCR PSTVd160 plasmid the Hind3/BamH1fill in PSTVd160 fragment was isolated to introduce it into the Hind3/Sal1fill in sites of pPCV702SM resulting in pPCV702SM PSTVd160. The final pPCV702SM GFP160 vector was obtained by insertion of the GFP BamH1/Sac1trimmed end cDNA fragment into the BamH1/Bgl2fill in sites of the pPCV702SM PSTVd160 plasmid (Fig. 7).

Primer sequences:

Bgl67-H: 5'-ttagatctccgctttttctc-3' (SEQ ID NO: 4)

PV30-H: 5'-tgaaccacaggaaccacgaggggg-3' (SEQ ID NO: 5)

PV175-H: 5'-tcctgtcggccgctgggcactccc-3' (SEQ ID NO: 6)

### Example 3: Plant transformation

The recombinant vectors pPCV702SM GFP100 and pPCV702SM GFP160 were introduced into N. tabacum SR1 plants according to the leaf-disk-transformation procedure. Independent transformants were regenerated, the completeness of non-rearranged T-DNA integration was shown by Southern analysis, and GFP expression was documented by taking photographs under UV-light and by Northern analysis (Fig. 4, lane 1 and 3).
Transgenic plant lines carrying a single copy of the GFP100 (SR1 GFP100) and GFP160 transgene constructs (SR1 GFP160) were self-fertilised and progeny of this genetic crosses were screened for homozygous lines (GFP100+/+, respectively, GFP160+/+) by Southern analysis. The homozygous state of the screened plant lines was confirmed by back-crosses to SR1 wild-type plants.

### Example 4: PSTVd Infection

Infection of the transgenic SR1GFP, SR1GFP100, and SR1GFP160 plant lines with PSTVd was either achieved by mechanical inoculation with total RNA isolated from a PSTVd-infected N. tabacum plant (Wassenegger et al., 1996) or by genetic crosses with a homozygous plant of the transgenic SR1-4(-) line (Wassenegger et al., 1994a). Infection was confirmed by Northern analysis as described in Wassenegger et al. (1994a).

### Example 5: GFP expression analysis

The SR1GFP was not affected by the PSTVd infection. Figure 8 shows an example of a progeny plant of the SR1GFPxSR1-4(-) cross photographed under UV-light. Because wild-type and non-GFP-expressing plants appear to be red-coloured under the UV-light conditions chosen, the green colour of SR1GFP-4(-) indicates clear expression of the GFP transgene. In figure 4, Northern analysis of this plant (lane 6) and of the PSTVd-free SR1GFP parental plant (lane 5) documents that the GFP is highly expressed in the leave tissue of both plants.
By contrast, PSTVd-infected SR1GFP100 and SR1GFP160 appeared to be red-coloured as wild-type SR1 plants under the same conditions. In addition, the steady-state GFP mRNA level of these plants was barely detectable by Northern analysis (Fig. 4, lane 2 and 4) whereas the corresponding PSTVd-free plants highly expressed the GFP (Fig. 4, lane 1 and 3). The difference between GFP expression in the PSTVd-infected SR1GFP100-4(-) plant lines and the PSTVd-free SR1GFP100 is further demonstrated by an UV-light photograph of a typical example of one of these plants (Fig. 9).
It was also found that similar silencing effects of the GFP transgene can be achieved by mechanical inoculation with total RNA isolated from an PSTVd-infected N. tabacum plant. UV-light photographs and Northern analysis confirmed that GFP mRNA was barely detectable in mechanically inoculated PSTVd-infected SR1GFP100 plant line.

### Example 6: DNA methylation analysis

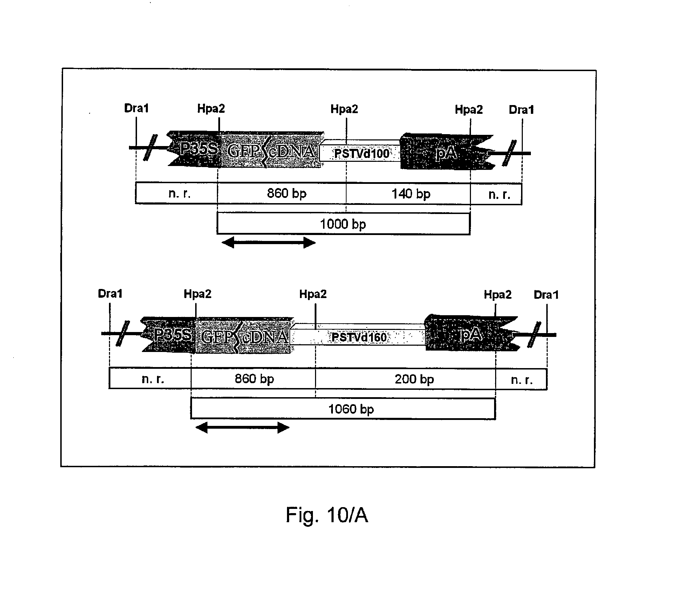

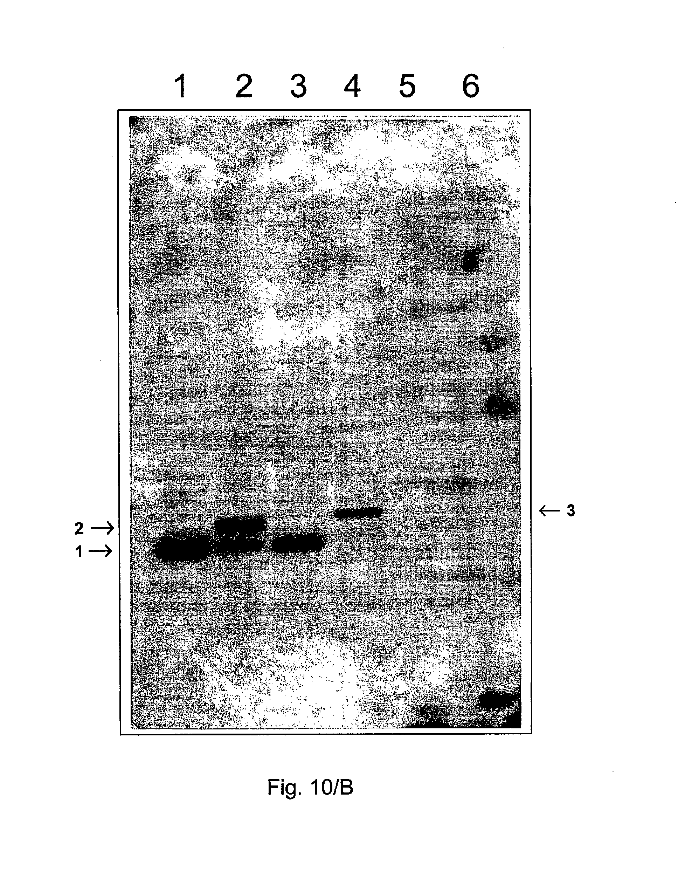

It was further demonstrated that specific de novo transgene methylation was only detectable in the genomic DNA from SR1GFP100 and SR1GFP160 plant lines that were PSTVd-infected. Figure 10/B shows the Southern analysis of a PSTVd-free SR1GFP100 (lane 1), a PSTVd-infected SR1GFP100-4(-) (lane 2), a PSTVd-free SR1GFP160 (lane 3), and a PSTVd-infected SR1GFP160-4(-) plant. The DNA of these plants was cut with Dra1 and with the methylation-sensitive restriction enzyme Hpa2. The shift of the hybridising GFP-specific fragment from 860 bp to 1000 bp (Fig. 10/B, lane 2) and from 860 bp to 1060 bp (Fig. 10/B, lane 4) shows that the Hpa2 enzyme failed to completely cut the genomic DNA of the two PSTVd-infected plants. This failure of restriction represented evidence for de novo methylation at the two Hpa2 sites tested (see also Fig. 10/A: physical map of the two transgenes).
The fact that production of specific siRNAs is induced upon PSTVd infection (Fig. 2) indicates that inactivation of the GFP100 and the GFP160 transgene underlies a PTGS-like mechanism (Fig. 11). Because RdDM and initiation of PTGS both require PSTVd infection, it can not be determined whether de novo DNA methylation contributes to the silencing process. The inventors previous work presented evidence that PSTVd RNA-directed DNA methylation was highly specific and sequences that were targeted for methylation only comprised the regions of homology between the target DNA and the directing RNA (Wassenegger, 2000). However, it can not be ruled out that the increased methylation of the genome-integrated transgenes leads to production of aberrant RNA (abRNA). The abRNA, in turn, is proposed to initiate PTGS as it may serve as template for the cellular RNA-directed RNA polymerase to produce dsRNA (Voinnet et al., 1998) (Fig. 12).

### Example 7: Trans-inactivation of GFP

In order to examine whether the inactivation of the GFP100 and GFP160 can trigger trans-inactivation, the SR1GFP100-4(-) and SR1GFP160-4(-) plants are crossed with SR1GFP plants. UV-light photographs and Northern analysis of progeny plants show whether the siRNAs produced in the SR1GFP100-4(-) and SR1GFP160-4(-) plant lines can also target the mRNA of the GFP transgene. First indications for such a mechanism are obtained by characterisation of the SR1 GFP100-4(-)-specific siRNAs.

### References:

Elbashir S.M., Lendeckel W., and Tuschl T. (2001). RNA interference is mediated by 21- and 22-nucleotide RNAs. Genes Dev. 15: 188-200
Fire A., Xu S.Q., Montgomery M.K., Kostas S.A., Driver S.E., and Mello C.C. (1998). Potent and specific genetic interference by double-stranded RNA in Caenorhabditis elegans. Nature 391: 806-811
Hamilton A.J. and Baulcombe D.C. (1999). A species of small antisense RNA in posttranscriptional gene silencing in plants. Science 286: 950-952
Hammond S.M., Caudy A.A., and Hannon G. (2001). Post-transcriptional gene silencing by double-stranded RNA. Nature Rev Genet 2: 110-119
Horsch R.B., Fry J.E., Hoffman N.L., Eichholz D., Rogers S.G., Fraley R.T. (1985). A simple and general method for transfering genes into plants. Science 227: 1229-1231.
Mette F., van der Winden J., Matzke M.A., and Matzke A.J.M. (1999). Production of aberrant promoter transcripts contributes to methylation and silencing of unlinked homologous promoters in trans. EMBO J 18: 241-248
Mette F., Aufsatz W., van der Winden J., Matzke M.A., and Matzke A.J.M. (2000). Transcriptional silencing and promoter methylation triggered by double-stranded RNA. EMBO J 19: 5194-5201
Papaefthimiou I., Hamilton, A.J., Denti M.A., Baulcombe D.C., Tsagris M., and Tabler M. (2001). Replicating potato spindle tuber viroid RNA is accompanied by short RNA fragments that are characteristic of post-transcriptional gene silencing. Nucl Acids Res 29: 2395-2400
Pélissier T., Thalmeir S., Kempe D., Sänger H.L., and Wassenegger M (1999). Heavy de novo methylation at symmetrical and non-symmetrical sites is a hallmark of RNA-directed DNA methylation. Nucl Acids Res 27: 1625-1634
Pélissier T. and Wassenegger M. (2000). A 30 bp homology is sufficient to trigger RNA-directed DNA methylation. RNA 6: 55-65
Sänger H.L. (1987). Viroid replication. In: The Viroids, T. O. Diener, ed. (New York: Plenum Press), pp. 117-166
Sambrook J., Fritsch E.F., and Maniatis T. (1989). Molecular cloning: A laboratory Manual, Second edition (Cold Spring Harbor, New York: Cold Spring Harbor Laboratory Press).
Stam M., Viterbo A., Mol J.N.M., and Kooter J.M. (1998). Position-dependent methylation and transcriptional silencing of transgenes in inverted T-DNA repeats: Implications for posttranscriptional silencing of homologous host genes in plants. Mol Cell Biol 18: 6165-6177
Tuschl T. (2001). RNA Interference and small interfering RNAs. Chembiochem 2: 239-245
Voinnet O., Vain P., Angell S., and Baulcombe, D.C. (1998). Systemic spread of sequence-specific transgene RNA degradation in plants is initiated by localized introduction of ectopic promoterless DNA. Cell 95: 177-187
Wang M.-B., Wesley S.W., Finnegan E.J., Smith N.A., and Waterhouse P.M. (2001). Replicating satellite RNA molecules induces sequence-specific DNA methylation and truncated transcripts in plants. RNA 7: 16-17
Wassenegger M., Heimes S., Riedel L., and Sänger H.L. (1994a). RNA-directed de novo methylation of genomic sequences in plants. Cell 76: 567-576
Wassenegger M., Heimes S., and Sänger H.L. (1994b). An infectious viroid RNA replicon evolved from an in vitro-generated non-infectious viroid deletion mutant via a complementary deletion in vivo. EMBO J 13: 6172-6177
Wassenegger M., Spieker R.L., Thalmeir S., Gast F.-U., Riedel L., and Sänger H.L. (1996). A single nucleotide substitution converts potato spindle tuber viroid (PSTVd) from a noninfectious to an infectious RNA for Nicotiana tabacum. Virology 226: 191-197
Wassenegger M. (2000). RNA-directed DNA methylation. Plant Mol Biol 43: 203-220
Wassenegger M. (2001). Advantages and disadvantages of using PCR techniques to characterize transgenic plants. Mol Biotech 17: 73-82
Waterhouse P.M., Graham M.W., Wang M.B. (1998). Virus resistance and gene silencing in plants can be induced by simultaneous expression of sense and antisense RNA. Proc Natl Acad Sci USA 95: 13959-13964

## Claims

1. A DNA construct comprising a DNA which can be transcribed in a plant cell to an RNA transcript, which RNA transcript includes a viroid sequence linked to a target gene sequence.

2. The DNA construct of claim 1, wherein said viroid sequence has a size of at least 30 base pairs (bp).

3. The DNA construct of claim 1 or 2 comprising a promoter operably linked to said DNA.

4. The DNA construct of any one of claims 1 to 3, wherein the viroid is potato spindle tuber viroid (PSTVd).

5. The DNA construct of any one of claims 1 to 4, wherein the target gene sequence is a cDNA sequence or part thereof.

6. A recombinant vector comprising a DNA construct of any one of claims 1 to 5.

7. A method of providing a plant for assessing the down-regulating expression of a target gene in a plant including the step of introducing a DNA construct or recombinant vector of any one of claims 1 to 6 into a plant cell preferably such that the construct is stably incorporated into the genome of the cell.

8. A plant obtainable by the method of claim 7.

9. A method of target gene modulation/characterization comprising infecting a plant of claim 8 with a plant viroid, and optionally comparing the phenotype of said plant to that of a corresponding wild type plant.

10. The method of claim 9 further comprising identifying and/or isolating the target gene or part thereof.
